# EUROPÄISCHE PATENTSCHRIFT

(11) **EP 0 794 767 B1**
(45) Veröffentlichungstag und Bekanntmachung des Hinweises auf die Patenterteilung: **22.07.1998**
(21) Anmeldenummer: 95942642.0
(22) Anmeldetag: 19.12.1995
(51) Int. Cl.: A61K 9/08, A61K 9/12, A61K 47/10, A61K 47/40

(54) **STABILE BUDESONID-LÖSUNGEN, VERFAHREN ZU DEREN HERSTELLUNG SOWIE VERWENDUNG DIESER LÖSUNGEN ALS KLISTIERZUBEREITUNGEN UND PHARMAZEUTISCHE SCHÄUME**
STABLE BUDESONIDE SOLUTIONS, METHOD OF PREPARING THEM AND USE OF THESE SOLUTIONS AS ENEMA PREPARATIONS AND PHARMACEUTICAL FOAMS
SOLUTIONS DE BUDESONIDE STABLES, LEUR PROCEDE DE PREPARATION ET LEUR UTILISATION COMME PREPARATIONS POUR LAVEMENT ET MOUSSES PHARMACEUTIQUES

(30) Priorität: 27.12.1994 DE 4446891
(43) Veröffentlichungstag der Anmeldung: 17.09.1997
(73) Patentinhaber: DR. FALK PHARMA GMBH, D-79108 Freiburg (DE)
(72) Erfinder: OTTERBECK, Norbert, 88662 Überlingen (DE); KÜHN, Reimund, 79348 Freiamt (DE)
(74) Vertreter: Keller, Günter, Dr.
(86) Internationale Anmeldenummer: DE9501847
(87) Internationale Veröffentlichungsnummer: WO9619969

(56) Entgegenhaltungen:
- EP-A- 0 468 555
- WO-A-94/16710
- WO-A-95/14474
- US-A- 4 383 992
- ATEMWEGS- UND LUNGENKRANKHEITEN, Bd. 20, Nr. 7, 1994, Seiten 381-382, XP000568623 J.DERBACHER ET AL.: "Physikalische Eigenschaften von Inhalationslösungen"
- J.PHARM.SCI., Bd. 84, Nr. 6, 1995, Seiten 677-681, XP000567853 H.NOLEN ET AL.: "Budesonide - beta-D-glucuronide: A potential Prodrug for Treatment of Ulcerative Colitis"

## Beschreibung

Die vorliegende Erfindung betrifft stabile Budesonid-Lösungen, Verfahren zu deren Herstellung sowie deren Verwendung zur Herstellung von Arzneimittelzubereitungen, insbesondere von Klistieren und pharmazeutischen Schäumen.

Budesonid (INN; 16α,17-Butylidendioxy-1β,21-dihydroxy-1,4-pregnadien-3,20-dion) ist ein bekannter Wirkstoff der Cortikoidreihe, der vor allem zur Behandlung von Bronchialerkrankungen, aber auch bei entzündlichen Darmerkrankungen, wie Morbus Crohn und insbesondere Colitis ulcerosa, eingesetzt wird. Bei der zuletzt genannten Indikation hat sich die Verabreichung von rektalen Arzneiformen, wie Klistierzubereitungen oder pharmazeutischen Schäumen in Druckgaspackungen als besonders geeignet erwiesen, da der Wirkstoff direkt am Ort der Erkrankung zum Einsatz gebracht wird und Budesonid vor allem topisch wirksam ist.

Budesonid stellt ein Razemat dar, bestehend aus einer Mischung der beiden Diastereomere 22R und 22S. Im Rahmen der vorliegenden Erfindung kann das Razemat eingesetzt werden, bevorzugt wird jedoch das 22R-Diastereomer eingesetzt, da dieses etwa um den Faktor 2-3 in pharmakologischer Hinsicht aktiver ist. Verfahren zur Auftrennung der Enantiomere sind bekannt, beispielsweise aus der EPA 92.901023.9.

Aufgrund seiner Lipophilie ist Budesonid in Wasser praktisch unlöslich, jedoch gut löslich in Alkoholen. Durch die Verwendung von Lösungsvermittlern wie organische wasserlösliche Alkohole kann eine ausreichende Menge Wirkstoff in Lösung gebracht werden. Die so erhaltenen Lösungen erweisen sich jedoch für die pharmazeutische Verwendung als zu wenig stabil, da innerhalb kurzer Zeit große Mengen des Wirkstoffes zersetzt sind.

Aufgrund dieser Instabilität sind Budesonid-Zubereitungen, die vom Patienten in der gebrauchsfertigen Darreichungsform direkt angewendet werden können, nicht bekannt. Zwar werden derzeit in einigen Ländern budesonidhaltige Klistiere angeboten, in diesen Fällen handelt es sich jedoch nicht um gebrauchsfertige Klistiere, sondern um eine Art Kombination aus wirkstoffhaltigen Tabletten und mit Wasser gefüllten Klistierflaschen. Vor Verabreichung muß der Patient jeweils eine Tablette entnehmen, sie in die geöffnete Klistierflasche einbringen, warten, bis die Tablette zerfallen ist und die Flaschen vor Gebrauch kräftig schütteln, um eine möglichst homogene Verteilung des Wirkstoffs in Form einer Suspension zu erreichen.

Diese mühsame und umständliche Zubereitung des gebrauchsfertigen Klistiers kann insbesondere von gebrechlichen Patienten nur unzureichend bewältigt werden. Eine homogene Verteilung des Wirkstoffs kann mit diesem Verfahren nur unzureichend erzielt werden und eine vollständige Verabreichung des Wirkstoffs durch Ausdrücken der Klistierflasche kann kaum garantiert werden, da die einmal zubereitete Endform in kurzer Zeit zur Absetzung des suspendierten Wirkstoffes am Boden der Flasche neigt.

Die rektale Verabreichung von Budesonid mittels Rektalschäumen hat zwar im Hinblick auf die Bequemlichkeit der Anwendung Vorteile, jedoch besteht hier das Problem, Budesonid-Lösungen zur Verfügung zu stellen, die ausreichend stabil sind, um in Druckgaspackungen appliziert zu werden. Daher sind budesonidhaltige Rektalschäume bisher nicht bekannt.

Budesonidlösungen des pH-Werts 3-4 sind in Atemwegs- und Lungenerkrankungen, Band 20, Nr. 7, 1994, Seiten 381-82 beschrieben. Desonidlösungen des pH-Werts 2.5-6 sind in WO-A-9416710 beschrieben.

Eine Aufgabe der vorliegenden Erfindung ist es, ausreichend stabile Lösungen von Budesonid bereitzustellen.

Gelöst wird diese Aufgabe durch Budesonid-Lösungen, die einen pH-Wert von 6 oder darunter aufweisen, bei der das Budesonid gelöst ist in Wasser, Alkohol oder einer Wasser/Alkohol-Mischung, wobei es sich bei dem Alkohol um Ethanol, Isopropanol oder Propylenglycol handelt.

Es wurde völlig überraschend gefunden, daß die Stabilität von budesonidhaltigen Lösungen entscheidend vom pH-Wert abhängig ist. Die Stabilität der Lösungen steigt mit abnehmendem pH-Wert an. Die erfindungsgemäßen Budesonid-Lösungen können auf wäßriger und/oder alkoholischer Grundlage hergestellt werden, wobei es auch möglich ist, Budesonid in einer wäßrigen, alkoholischen Mischung zu lösen.

Im Rahmen der vorliegenden Erfindung wurde völlig überraschend herausgefunden, daß der pH-Wert einen entscheidenden Einfluß auf die Stabilität der Budesonid-Lösung hat, und zwar in wäßriger, wäßrig-alkoholischer oder alkoholischer Lösung.

Die nachfolgend wiedergegebene Tabelle 1 belegt die Stabilität einer wäßrigen Lösung von Budesonid und die Tabelle 2 zeigt die Stabilität einer alkoholischen Lösung (Propylenglycol) von Budesonid in Abhängigkeit von der Lagerzeit und dem pH-Wert.

**Tabelle 1**

| Stabilität von erfindungsgemäßen Budesonid-Lösungen (2 mg/60 ml H₂O) in Abhängigkeit von der Lagerzeit | | |
|---|---|---|
| pH | Gehalt¹⁾ nach 14 Tagen Lagerung bei | |
| | 20°C | 40°C |
| 7,0 | 96,5 % | 71,2 % |
| 6,5 | 96,4 % | 77,1 % |
| 6,0 | 97,6 % | 84,8 % |
| 5,5 | 97,4 % | 87,6 % |
| 5,0 | 97,6 % | 90,0 % |
| 4,5 | 97,3 % | 93,0 % |
| 4,0 | 98,7 % | 95,2 % |
| 3,5 | 99,8 % | 96,6 % |
| 3,0 | 100,0 % | 97,9 % |

| | | |
|---|---|---|
| ¹⁾ Ausgangswert nach t = 0 Tagen gleich 100 % | | |

Aus der vorstehenden Tabelle 1 geht hervor, daß die Stabilität der Lösungen mit abnehmendem pH-Wert steigt. Selbst nach 14tägiger Lagerung bei 20°C und pH 3,0 ist der Wirkstoff noch zu 100 % vorhanden. Auch bei einer Lagerung bei einer deutlich erhöhten Temperatur, d.h. bei 40°C und pH 3,0, befinden sich nach 14 Tagen noch 97,9 % Budesonid in der Lösung.

**Tabelle 2**

| Stabilität von erfindungsgemäßen Budesonid-Lösungen (2 mg/4 g in Propylenglycol) in Abhängigkeit von der Lagerzeit Gehalt nach Lagerung bei | | | | |
|---|---|---|---|---|
| **pH** | **0 Monate** | **3 Monate 25°C** | **3 Monate 40°C** | **6 Monate 40°C** |
| 7,5 | 100,0 % | 80,6 % | 44,4 % | 15,3 % |
| 5,0 | 100,0 % | 103,0 % | 96,5 % | 89,5 % |
| 2,8 | 100,0 % | 99,4 % | 98,7 % | 95,2 % |

Aus der vorstehenden Tabelle 2 geht hervor, daß die Stabilität der Lösungen mit abnehmendem pH-Wert ansteigt.

Ab pH-Werten von 4,0 und tiefer werden ausreichend niedrige Wirkstoffverluste erhalten, die in Abhängigkeit von der Lagerzeit in einer für pharmazeutische Produkte akzeptablen Größenordnung liegen.

Für die pharmazeutische Verwendung sind bei der Anwendung als Klistier- oder auch als Rektalschaum aufgrund physiologischer Gegebenheiten und unter Berücksichtigung der zu verabreichenden Menge von ca. 30 bis 100 ml für Klistiere bzw. von etwa 4 g bei Rektalschäumen pH-Werte der gebrauchsfertigen Form von ≥ 3,5 zu bevorzugen.

Für die Einstellung des pH-Wertes können beliebige pharmazeutisch annehmbare organische und anorganische Säuren verwendet werden, beispielsweise Salzsäure, Phosphorsäure, Zitronensäure oder Weinsäure.

Um die Stabilität weiter zu erhöhen, z.B. zur Einsparung von Transport- und Lagerkosten oder auch bei Verwendung als nicht rektale Applikationsform, können auch konzentrierte Budesonid-Lösungen mit einem pH-Wert von ≤ 3,5 hergestellt werden. Falls für die spätere Verwendung eine Einstellung des pH-Wertes auf einen physiologisch verträglichen Wert von > 3,5 notwendig ist, so kann dies erst kurz vor der Anwendung erfolgen. Dies kann z.B. durch Verdünnen oder durch Zugabe einer Base geschehen. Durch den Verdünnungsvorgang erhöht sich dann der pH-Wert.

In weiteren bevorzugten Ausführungsformen weisen die erfindungsgemäßen Budesonid-Lösungen einen Zusatz von Natrium-EDTA (Natrium-Ethylendiamintetraessigsäure; Komplexon) auf, was zu einer weiteren Erhöhung der Stabilität führt.

In einer anderen bevorzugten Ausführungsform der vorliegenden Erfindung kann die Stabilität der Lösung durch die Verwendung von Cyclodextrinen, vorzugsweise von Hydroxypropyl-β-cyclodextrin oder γ-Cyclodextrin erhöht werden. Der Zusatz von Cyclodextrinen ermöglicht auch die Verwendung von konzentrierteren Lösungen von Budesonid.

Gegenstand der Erfindung sind daher stabile Budesonid-Lösungen mit einem pH-Wert von 6,0 oder darunter, wobei das Budesonid in Wasser, Alkohol oder einer Wasser/Alkohol-Mischung gelöst ist, und als Alkohole Propylenglycol, Ethanol oder Isopropanol verwendet, werden.

Wenn eine Alkohol/Wasser-Mischung eingesetzt wird, liegt das Verhältnis Alkohol zu Wasser zwischen 100:0 und 80:20, bevorzugt zwischen 98:2 und 93:7.

Der Gehalt an Budesonid in der gebrauchsfertigen Lösung liegt zwischen 0,001 und 1 Gew.-%, bevorzugt zwischen 0,01 und 0,1 Gew.-% und bei Klistieren besonders bevorzugt bei 0,001 bis 0,1 Gew.-%.

Die erfindungsgemäßen Lösungen können ferner Hilfsstoffe enthalten, wie sie üblicherweise bei den entsprechenden pharmazeutischen Formulierungen verwendet werden. Diese Hilfsstoffe können zur Lösungsvermittlung von Cortikoiden geeignet sein. Derartige Hilfsstoffe sind dem Fachmann geläufig.

Übliche Hilfsstoffe sind solche, die die Viskosität der Lösung beeinflussen, üblicherweise erhöhen, Konservierungsmittel wie Ethanol, Chlorbutanol, Benzylalkohol, Phenylethanol, Sorbinsäure, Benzoesäure, Natriumdisulfit, p-Hydroxybenzoesäureester, Phenol, m-Kresol, p-Chlor-m-Kresol, Quats, Chlorhexidin, Verdickungsmittel wie Gelatine, Tragant, Pektin, Cellulosederivate (z.B. Methylcellulose, Hydroxypropyl-Methylcellulose, Carboxymethylcellulose-Natrium), Polyvinylpyrrolidon, Polyvinylalkohol, Polyacrylsäuren, Xanthangummi, Säuren wie Essigsäure, Zitronensäure, Weinsäure, Salzsäure, Phosphorsäure; Basen wie Kaliumhydroxid, Natriumhydoxid; Puffersubstanzen wie Salzsäurepuffer, Phthalatpuffer, Phosphatpuffer, Boratpuffer, Acetatpuffer oder Citratpuffer. Um die Löslichkeit des Wirkstoffes zu erhöhen, eignen sich z.B. der Zusatz von ausreichenden Mengen von Alkoholen wie Ethanol, Isopropanol, Glycerin, Propylenglycol, Polyethylenglycole oder der Einsatz von Lösungsvermittlern wie z.B. Cyclodextrinen, vorzugsweise β-Cyclodextrin, Hydroxypropyl-β-Cyclodextrin und/oder γ-Cyclodextrin.

Wenn die erfindungsgemäßen Budesonid-Lösungen für Rektalschäume verwendet werden sollen, müssen solche üblichen Hilfsstoffe zugegeben werden, die die zu bildende Dispersion erst ermöglichen. Bei derartigen Hilfsstoffen handelt es sich um dem Fachmann bekannte Emulgatoren wie Emulgine und verschiedene Lanettetypen. Weiterhin können Konservierungsmittel wie Sorbinsäure, Parahydroxybenzoesäureester, Benzoesäure, Säuren wie Essigsäure, Zitronensäure, Weinsäure, Salzsäure und Phosphorsäure zugegeben werden.

Bei Rektalschäumen werden in die Druckpackungen auch die geeigneten Treibgase gegeben. Auch wenn Chlorfluorkohlenwasserstoffgase als Treibgase geeignet wären, sieht man derzeit von der Verwendung dieser Gase aus Umweltschutzgründen eher ab. Bevorzugt werden daher Kohlenwasserstoffe wie Isobutan, n-Butan oder Gemische aus Propan/n-Butan als Treibgase verwendet.

Der pH-Wert der erfindungsgemäßen Budesonid-Lösungen liegt bei 6,0 oder darunter. Bevorzugt ist ein Bereich zwischen 5,0 und 3,5 und der pH-Bereich zwischen 4,5 und 4,0 ist besonders bevorzugt.

In den erfindungsgemäßen Lösungen ist Budesonid in einer Menge zwischen 0,001 bis 1 Gew.-% vorhanden, wobei die Konzentration bei Lösungen, die als Klistiere verwendet werden sollen, niedriger ist als bei Lösungen, die für Rektalschäume vorgesehen sind. Bei Lösungen für Klistiere beträgt die bevorzugte Konzentration zwischen 0,01 und 0,1 Gew.-% und bei Rektalschäumen liegt der bevorzugte Bereich zwischen 0,01 und 0,1 Gew.-%.

Die Zugabe von Natrium-EDTA erfolgt bevorzugt in einer Menge von 0,01 bis 1,0 Gew.-%, wobei bei Klistieren ein Bereich von 0,01 bis 0,1 Gew.-% und bei Rektalschäumen eine Konzentration von 0,05 bis 1 Gew.-% bevorzugt ist.

Sofern Cyclodextrine als Lösungsvermittler zugegeben werden, erfolgt dies bevorzugt in einer Menge zwischen 0,05 und 0,5 Gew.-%, wobei eine Zugabe von etwa 0,1 Gew.-% bevorzugt ist.

Die Herstellung von Lösungen für Klistiere erfolgt nach an sich bekannten Verfahren. Es kann beispielsweise eine ethanolische Stammlösung von Budesonid einer wäßrigen Lösung der weiteren Bestandteile unter Homogenisierung eingearbeitet werden. Die Verwendung eines Oxidationsschutzmittels bzw. der Ausschluß von Sauerstoff sowie Maßnahmen im Bereich des Lichtschutzes sind nicht speziell erforderlich, sie können jedoch die Qualität und auch die Stabilität der erhaltenen Produkte verbessern. Für die rektale Anwendung wird dann die fertige Lösung in eine übliche flexible Klistierflasche mit Applikationsspitze abgefüllt, die beide bevorzugt aus opakem Kunststoff hergestellt sind.

Auch die Herstellung von Lösungen für Rektalschäume ist aus dem Stand der Technik bekannt. Beispielsweise kann das Konservierungsmittel sowie die zur Schaumbildung benötigten Emulgatoren in der entsprechenden Lösung, bevorzugt dem geeigneten Alkohol gelöst werden. Danach wird der Wirkstoff als alkoholische Stammlösung in diese Lösung eingearbeitet. Im letzten Schritt werden Komplexon und die entsprechende Säure, gelöst in einer kleinen Menge Wasser, unter Homogenisieren in die alkoholische Lösung eingerührt.

Wenn die erfindungsgemäße Budesonid-Lösung zur Herstellung eines Rektalschaumes verwendet wird, wird die fertige Lösung in geeignete Druckgaspackungen, die mit kommerziell erhältlichen Ventilsystemen als Einmal- oder Mehrfachdosierer versehen sind, abgefüllt und mit einem Treibgas versetzt. Die Packungen enthalten zusätzlich eine Applikatorspitze aus Kunststoff. Aufgrund der chemischen und physikalischen Eigenschaften der erfindungsgemäßen stabilen Budesonid-Lösung wird bei der Applikation zwangsläufig der Schaum im Rektum erzeugt.

In bevorzugter Ausführungsform werden die erfindungsgemäßen stabilen Budesonid-Lösungen zur Herstellung von Klistieren oder Rektalschäumen verwendet. Andere Verwendungsformen wie beispielsweise Verwendung als Dosieraerosol, Inhalationsspray oder auch als Tropfen, Sirup oder Elixiere sind ebenfalls möglich und können von dem Fachmann auf der Basis der vorliegenden Beschreibung leicht hergestellt werden. Die Erfindung wird anhand der folgenden Beispiele näher erläutert.

### Beispiel 1

Nach dem vorstehend beschriebenen Verfahren wird eine Lösung hergestellt und in entsprechende Kunststoffflaschen abgefüllt, die pro 60 g Klistierflasche folgende Bestandteile enthält:

| | |
|---|---|
| Budesonid | 2 mg |
| β-Cyclodextrin, Hydroxypropyl-β-cyclodextrin (0,9) bzw. γ-Cyclodextrin | 60 mg |
| Natrium-EDTA | 30 mg |
| Natriumbenzoat | 300 mg |
| Xanthangummi | 360 mg |
| 2N HCl ad pH 4,0 | |
| Ethanol | 400 mg |
| Wasser ad | 60,00 mg |

Die so hergestellte Lösung erwies sich selbst nach mehrwöchiger Lagerung bei 40°C als stabil.

### Beispiel 2

Klysma, hergestellt nach üblichem Verfahren, enthaltend

| | |
|---|---|
| Budesonid | 2 mg |
| Natriumbenzoat | 300 mg |
| Natrium-EDTA | 30 mg |
| Propylenglycol | 500 mg |
| Xanthangummi | 360 mg |
| 2H Salzsäure ad pH 3,5 | 60,00 mg |

Das so hergestellte Klysma erwies sich nach mehrwöchiger Lagerung bei 40°C als stabil.

### Beispiel 3

Gemäß Beispiel 1 wird eine wäßrige Lösung hergestellt, die die folgenden Bestandteile enthält:

| | |
|---|---|
| Budesonid | 2 mg |
| β-Cyclodextrin, Hydroxypropyl-β-cyclodextrin (0,9) bzw. γ-Cyclodextrin | 60 mg |
| Natriumbenzoat | 300 mg |
| Xanthangummi | 360 mg |
| 2N HCl ad pH 4,0 | |
| Ethanol | 400 mg |
| Wasser ad | 60,00 mg |

Die so hergestellte Lösung erwies sich selbst nach mehrwöchiger Lagerung bei Raumtemperatur als stabil.

### Beispiel 4

Gemäß Beispiel 1 wird eine wäßrige Lösung hergestellt, die die folgenden Bestandteile enthält:

| | |
|---|---|
| Budesonid | 2 mg |
| Natrium-EDTA | 30 mg |
| Natriumbenzoat | 300 mg |
| Xanthangummi | 360 mg |
| 2N HCl ad pH 4,0 | |
| Ethanol | 400 mg |
| Wasser ad | 60,00 mg |

Die so hergestellte Lösung erwies sich selbst nach mehrwöchiger Lagerung bei Raumtemperatur als stabil.

### Beispiel 5

Gemäß Beispiel 1 wird eine wäßrige Lösung hergestellt, die die folgenden Bestandteile enthält:

| | |
|---|---|
| Budesonid | 2 mg |
| Natriumbenzoat | 300 mg |
| Xanthangummi | 360 mg |
| 2N HCl ad pH 4,0 | |
| Ethanol | 400 mg |
| Wasser ad | 60,00 mg |

Die so hergestellte Lösung erwies sich selbst nach mehrwöchiger Lagerung bei Raumtemperatur als stabil.

### Beispiel 6

### Untersuchung zur Stabilität der erfindungsgemäßen Lösungen

Es wurden gemäß Beispiel 1 die Formulierungen der folgenden Tabelle 3 hergestellt und auf ihre Stabilität nach 1- bis 39-wöchiger Lagerung untersucht. Es wurden die folgenden Ergebnisse erhalten.

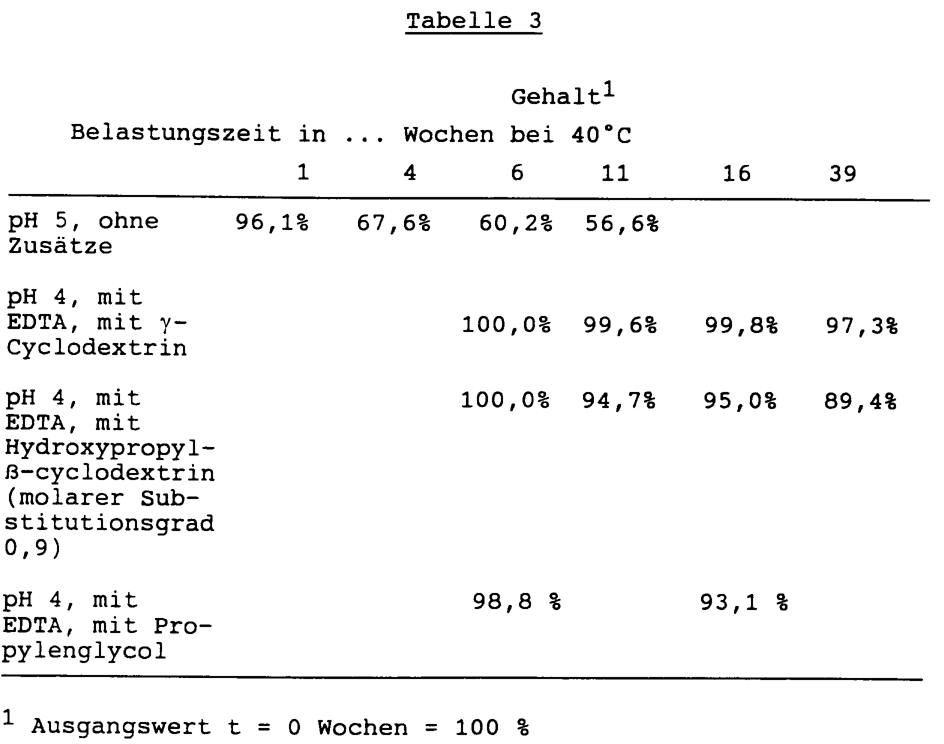

Die vorstehenden Versuchsergebnisse belegen, daß die erfindungsgemäßen wäßrigen Budesonid-Lösungen mit einem pH-Wert unter 6 auch nach mehrwöchiger Lagerung immer noch stabil sind. Besonders stabil sind Lösungen, die EDTA und ein Cyclodextrin enthalten. So liegen in diesen Lösungen selbst nach sechswöchiger Lagerung bei 40°C und bei einem pH von 4 immer noch 100 % Wirkstoff vor.

### Beispiel 7

Nach dem vorstehend beschriebenen Verfahren wird eine Lösung hergestellt und in entsprechende Druckgaspackungen (z.B. Aluminiummonoblockdose, 55 ml, mit Flüssigkeitsventil, Shield und Dosierkopf) unter Zusatz eines Treibgases (Druck > 1,5 bar, wie z.B. Isobutan, n-Butan, Gemische aus Propan/n-Butan) abgefüllt.

Die Lösung enthält pro 35 g folgende Bestandteile:

| | |
|---|---|
| Budesonid | 0,01820 g |
| Lanette 0 | 0,45500 g |
| Eumulgin B1 | 0,09100 g |
| Eumulgin B2 | 0,09100 g |
| Sorbinsäure | 0,00845 g |
| Komplexon | 0,03000 g |
| Citronensäure ad pH 3,5 | 0,01000 g |
| Aqua purificata | 0,90000 g |
| Propylenglykol ad | 35,0000 g |

Die so hergestellte Lösung erwies sich nach mehrmonatiger Lagerung bei 40°C als stabil.

### Beispiel 8

| | |
|---|---|
| Budesonid | 0,01820 g |
| Lanette O | 0,45500 g |
| Eumulgin B1 | 0,09100 g |
| Eumulgin B2 | 0,09100 g |
| Benzoesäure | 0,06000 g |
| Citronensäure ad pH 4,0 | q.s. |
| Aqua purificata | 0.90000 g |
| Propylenglykol ad | 35.0000 g |

Die so hergestellte Lösung erwies sich nach mehrmonatiger Lagerung bei 40°C als stabil.

### Beispiel 9

| | |
|---|---|
| Budesonid | 0,01820 g |
| Lanette 0 | 0,45500 g |
| Eumulgin B1 | 0,09100 g |
| Eumulgin B2 | 0,09100 g |
| Komplexon | 0,03000 g |
| Weinsäure ad pH 4,5 | q.s. |
| Aqua purificata | 0,90000 g |
| Propylenglykol ad | 35.0000 g |

Die so hergestellte Lösung erwies sich nach mehrmonatiger Lagerung bei 40°C als stabil.

### Beispiel 10

### Untersuchungen zur Stabilität der erfindungsgemäßen Lösungen

Es wurden Formulierungen gemaß Beispiel 7 mit unterschiedlichen pH-Werten hergestellt und auf ihre Stabilität nach 0 - 6-monatiger Lagerung untersucht. Folgende Ergebnisse wurden erhalten:

**Tabelle 4**

| Gehalt nach Lagerung bei | | | | |
|---|---|---|---|---|
| pH | 0 Monate | 3 Monate 25°C | 3 Monate 40°C | 6 Monate 40°C |
| 7,5 ohne Zusätze | 100,0 % | 90,4 % | 75,0 % | nicht best. |
| 7,5 mit Komplexon | 100,0 % | 103,0 % | 96,5 % | 89,5 % |
| 4,5 (94073) mit Komplexon, mit Zitronensäure | 100,0 % | 102,5 % | 97,8 % | 94,6 % (9 Monate) |
| 4,0 (95030) mit Komplexon, mit Zitronensäure | 100,0 % | 97,6 % | 98,0 % | ― |
| 3,5 (95035) mit Komplexon, mit Zitronensäure | 100,0 % | 100,8 % | 100,2 % | -- |

Die vorstehenden Versuchsergebnises belegen, daß die erfindungsgemäßen alkoholischen Budesonid-Lösungen mit einem pH-Wert unter 6 auch nach mehrmonatiger Lagerung noch stabil sind. Besonders stabil sind Lösungen, die zusätzlich noch Komplexon enthalten. Selbst nach 6-9 Monaten bei 40°C-Lagerung sind die aufgetretenen Wirkstoffverluste äußerst gering.

## Patentansprüche

1. Stabile Budesonid-Lösung mit einem pH-Wert von 6 oder darunter, bei der das Budesonid gelöst ist in Wasser, Alkohol oder einer Wasser/Alkohol-Mischung, dadurch gekennzeichnet, daß es sich bei dem Alkohol um Ethanol, Isopropanol oder Propylenglycol handelt.

2. Stabile Budesonid-Lösung nach Anspruch 1, dadurch gekennzeichnet, daß sie zusätzlich Natrium-EDTA, Cyclodextrin oder Gemische davon enthält.

3. Stabile Budesonid-Lösung nach einem der vorhergehenden Ansprüche, dadurch gekennzeichnet, daß sie 0,001 bis 0,1 Gew.-% Budesonid enthält.

4. Stabile Budesonid-Lösung nach einem der vorhergehenden Ansprüche, dadurch gekennzeichnet, daß sie 0,01 bis 1 Gew.-% Natrium-EDTA und/oder 0,05 bis 1,0 Gew.-% Cyclodextrine enthält.

5. Stabile Budesonid-Lösung nach einem der vorhergehenden Ansprüche, dadurch gekennzeichnet, daß sie einen pH-Wert von unter 6 besitzt, 0,001 bis 0,1 Gew.-% Budesonid, 0,001 bis 0,1 Gew.-% Natrium-EDTA und/oder 0,05 bis 1,0 Gew.-% Cyclodextrine enthält.

6. Verfahren zur Herstellung von stabilen budesonidhaltigen Lösungen nach einem der vorhergehenden Ansprüche, dadurch gekennzeichnet, daß das Budesonid gelöst wird in Wasser, Alkohol oder einer Wasser/Alkohol-Mischung, wobei es sich bei dem Alkohol um Ethanol, Isopropanol oder Propylenglycol handelt und, daß der pH-Wert der Budesonid-Lösung auf einen Wert von 6,0 oder darunter eingestellt wird.

7. Klistier, dadurch gekennzeichnet, daß es eine stabile Budesonid-Lösung nach einem der Ansprüche 1 bis 5 umfaßt.

8. Rektalschaum, dadurch gekennzeichnet, daß dieser eine stabile wäßrige Budesonid-Lösung nach einem der Ansprüche 1 bis 5 umfaßt.

9. Verwendung einer stabilen Budesonid-Lösung nach einem der Ansprüche 1 bis 5 zur Herstellung eines Klistiers.

10. Verwendung einer stabilen Budesonid-Lösung nach einem der Ansprüche 1 bis 5 zur Herstellung eines Rektalschaumes.

## Claims

1. A stable budesonide solution with a pH of 6 or below, in which the budesonide is dissolved in water, alcohol or a water/alcohol mixture, characterized in that the alcohol is ethanol, isopropanol or propylene glycol.

2. A stable budesonide solution as claimed in claim 1, characterized in that it additionally comprises sodium EDTA, cyclodextrin or mixtures thereof.

3. A stable budesonide solution as claimed in any of the preceding claims, characterized in that it comprises 0.001 to 0.1 % by weight of budesonide.

4. A stable budesonide solution as claimed in any of the preceding claims, characterized in that it comprises 0.01 to 1 % by weight of sodium EDTA and/or 0.05 to 1.0 % by weight of cyclodextrins.

5. A stable budesonide solution as claimed in any of the preceding claims, characterized in that it has a pH below 6 and comprises 0.001 to 0.1 % by weight of budesonide, 0.001 to 0.1 % by weight of sodium EDTA and/or 0.05 to 1.0 % by weight of cyclodextrins.

6. A process for producing stable budesonide-containing solutions as claimed in any of the preceding claims, characterized in that the budesonide is dissolved in water, alcohol or a water/alcohol mixture, the alcohol being ethanol, isopropanol or propylene glycol, and that the pH of the budesonide solution is adjusted to a value of 6.0 or below.

7. An enema characterized in that it comprises a stable budesonide solution as claimed in any of claims 1 to 5.

8. A rectal foam characterized in that it comprises a stable aqueous budesonide solution as claimed in any of claims 1 to 5.

9. The use of a stable budesonide solution as claimed in any of claims 1 to 5 for producing an enema.

10. The use of a stable budesonide solution as claimed in any of claims 1 to 5 for producing a rectal foam.

## Revendications

1. Solution de budésonide stable, d'un pH de 6 ou inférieur à 6, dans laquelle le budésonide est dissous dans de l'eau, l'alcool, ou un mélange d'eau et d'alcool, caractérisée en ce que, dans le cas de l'alcool, il s'agit d'éthanol, d'isopropanol ou de propylèneglycol.

2. Solution de budésonide stable suivant la revendication 1, caractérisée en ce qu'elle contient complémentairement de l'EDTA sodique, de la cyclodextrine ou un mélange de ceux-ci.

3. Solution de budésonide stable suivant l'une quelconque des revendications précédentes, caractérisée en ce qu'elle contient de 0,001 à 0,1% en poids de budésonide.

4. Solution de budésonide stable suivant l'une quelconque des revendications précédentes, caractérisée en ce qu'elle contient de 0,01 à 1% en poids d'EDTA sodique et/ou de 0,05 à 1,0% en poids de cyclodextrines.

5. Solution de budésonide stable suivant l'une quelconque des revendications précédentes, caractérisée en ce qu'elle possède une valeur de pH inférieure à 6 et contient de 0,001 à 0,1% en poids de budésonide, de 0,001 à 0,1% en poids d'EDTA sodique et/ou de 0,05 à 1,0% en poids de cyclodextrines.

6. Procédé de préparation de solutions contenant du budésonide stable suivant l'une quelconque des revendications précédentes, caractérisé en ce que l'on dissous le budésonide dans de l'eau, de l'alcool ou un mélange d'eau et d'alcool, où il s'agit, dans le cas de l'alcool, d'éthanol, d'isopropanol ou de propylèneglycol et on règle la valeur du pH de la solution de budésonide à 6,0 ou à une valeur inférieure.

7. Lavement, caractérisé en ce qu'il comprend un solution de budésonide stable suivant l'une quelconque des revendications 1 à 5.

8. Mousse rectale, caractérisée en ce qu'elle comprend une solution aqueuse de budésonide stable suivant une quelconque des revendications 1 à 5.

9. Utilisation d'une solution de budésonide stable suivant l'une quelconque des revendications 1 à 5, en vue de la préparation d'un lavement.

10. Utilisation d'une solution de budésonide stable suivant l'une quelconque des revendications 1 à 5, en vue de la préparation d'une mousse rectale.
